Europäisches Patentamt

European Patent Office

Office européen des brevets

(11) Publication number: **0 395 757 B1**

## EUROPEAN PATENT SPECIFICATION

(45) Date of publication of patent specification: **15.06.94**  (51) Int. Cl.5: **A61K 35/78**

(21) Application number: **88907789.7**

(22) Date of filing: **13.09.88**

(86) International application number:
**PCT/JP88/00922**

(87) International publication number:
**WO 89/02444 (23.03.89 89/07)**

(54) **PROCESS FOR EXTRACTING PHYSIOLOGICALLY ACTIVE SUBSTANCE FROM PINE SEED SHELLS AND ANTIINFECTANT PREPARED MAINLY FROM THE EXTRACT.**

(30) Priority: **17.09.87 JP 233472/87**

(43) Date of publication of application:
**07.11.90 Bulletin 90/45**

(45) Publication of the grant of the patent:
**15.06.94 Bulletin 94/24**

(84) Designated Contracting States:
**BE CH DE FR GB IT LI NL SE**

(56) References cited:
**EP-A- 0 296 626**
**DE-C- 463 803**

**WPIL, FILE SUPPLIER, accession no. 87-294418, Derwent Publications Ltd, London, GB; & JP-A-62 205 032 (SAKAGAMI) 09-09-1987**

(73) Proprietor: **YOSHIHARA, Masazumi**
**1-12, Kawaramachi, Naka-ku**
**Hiroshima-shi, Hiroshima 733(JP)**

(72) Inventor: **YOSHIHARA, Masazumi**
**1-12, Kawaramachi**
**Naka-ku**
**Hiroshima-shi Hiroshima 733(JP)**
Inventor: **SAKAGAMI, Hiroshi**
**2-246, Chihusadai-danchi**
**33, Chigusadai**
**Midori-ku Yokohama-shi Kanagawa 227(JP)**

(74) Representative: **Wagner, Karl H. et al**
**WAGNER & GEYER**
**Patentanwälte**
**Gewürzmühlstrasse 5**
**D-80538 München (DE)**

**Description**

BACKGROUND OF THE INVENTION:

The present invention relates to a method of extracting a physiologically active acid polysaccharide from the husks of pine nuts such as black pine, red pine and white pine trees and an anti-contagion medicine in which the aforementioned extract is a principal component.

DESCRIPTION OF THE PRIOR ART:

For many years, the pine nuts have been considered to be an edible nut of high nutritive value.

In particular, a pine nut oil extracted from the pine nuts has been evaluated as a nutritious component having a physiological activity. Also in some districts pinecorns containing pine nuts therein have been evaluated by means of decoction for potential medical usage. In either case, it is well known that the pine nuts or the pinecorns contain some highly physiological active substances.

Assuming the aforementioned matters, the inventors of the present invention have carried out the development and research work in order to enlarge the scope of utilization of the pine nuts as well as the pinecorns. For instance, techniques to extract oil from pine nuts and techniques to extract the pine nuts from the firm husks of the pine nuts have been developed.

Extraction of pine nut oil resulted in a residue in the form of a pressed cake. To obtain the residue the husks are removed from the pine nuts and are decocted by means of boiling water (known as hot water extraction). The solution obtained by the extraction treatment undergoes paper filtration and is concentrated.

As a result of this experiment the inventors found a polysaccharide which has a remarkably high physiological activity. In particular, an activation function of granulocyte in a white blood corpuscle, is a great importance.

4. SUMMARY OF THE INVENTION:

It is an object of the present invention to efficiently extract from pine nut husks an acid polysaccharide with a remarkable activation function. For this purpose the husks of pine nuts are treated by fat removal treatment immersed in an alkaline solution for proceeding alkaline solution extract teatment and then the pH of the extracted solution is adjusted. The extracted solution is treated by a centrifugal separator and an extract is precipitated. The precipitated extract is concentrated by means of filtration treatment. The concentrated extract solution is made into a granular acid polysaccharide after lyophilization. Thus an essence contained in the husks of pine nuts is extracted and recovered.

In other words, the present invention is characterized in that the fats and other lower polymer substances are removed from the husks of the pine nuts and other lower polymer substances out of them by means of fat removal treatment. Then alkaline solution is added for proceeding an extraction. An extract solution obtained after extracting treatment is adjusted to have a certain pH. Thus an acid polysaccharide is extracted and recovered from the husks of the pine nuts.

As a result of various experiments, it was shown that the white pine nuts are the most preferable ones surpassing other black and red pine nuts for the purpose of the present invention.

It was also shown that the husks of the white pine nuts deforestated in the middle of November is most preferable.

The husks of the pine nuts are solid and heretofore it has been difficult to remove the husks efficiently from the pine nuts. However, the inventors have successfully invented a device including a breaking and crushing means and, as a result, removing the husks from the pine nuts is much easier. It was also shown that if the husks separated from the nuts are dried, and they are crushed to a degree suitable for extraction, the working efficiency of the present invention is greatly improved.

The alkaline solution which is used in the extraction treatment of the present invention is selected from some alkaline solutions. However, taking into consideration after-treatments of the extracted solution and extracted substance, ammonia water which can remove alkali by heat treatment is considered to be most preferable.

A degree of alkalization of the alkaline solution depends upon the sort or contents of the husks of pine nuts. It was shown that neutral or over neutral is desirable, and preferably, a degree between pH 7.5 to 10 is most desirable.

The extraction work of the present invention is started from a process of immersing the husks of pine nuts, which were broken and crushed to a desirable size, into an alkaline solution.

The amount of alkaline solution to be used for extraction is not particularly limited but it is preferable to use an amount as much as five times the husks of pine nuts. The alkaline solution of ordinary temperature is used but the alkaline solution of high temperature can also be used.

The extracted solution is separated from the husks of pine nuts by means of filtration treatment or the like, and it is concentrated to a proper degree of concentration by means of decompression, concentration, or the like. Then the pH is adjusted by means of a proper acid such as hydrochloric acid or the like. Finally a granular type acid polysaccharide is recovered through final lyophilization.

The granules of acid polysaccharide obtained and recovered by a process of the present invention have a distinct smell. The granules are brown in colour and are easily dissolved by water.

DETAILED DESCRIPTION OF THE PREFFERRED EMBODIMENTS:

The extraction method of acid polysaccharide is explained on the basis of the following preferred embodiments:

The pine nuts of the white pine tree collected at Konchianshi of Chiirin-sho in the People's Republic of China in the middle of November are prepared for treatment.

As a pretreatment of a process of the present invention, 1 kg of the husks of the pine nuts are washed with about four liters methanol solution. Oils and fats and lower polymer substances contained in the husks of pine nuts are removed.

Next, the husks of pine nuts washed in the above pretreatment are immersed in about four liters of boiling water. This hot water extracting treatment is repeated three times, each for six hours. The obtained crude extracted solution is filtered by paper filters and insoluble substances are removed. The obtained extracted solution is concentrated under the decompressed condition. The obtained concentrated solution is diluted with ethanol as much as six times the amount of the concentrated solution. The obtained precipitate is dissolved by distilled water, then lower polymer substances are removed through dialysis treatment, and finally the so-called hot water extract is recovered.

Further, six liters ammonia water of 0.8% conc. is added to the residue remaining after said hot water extraction treatment, and the mixture is stirred for three hours at 45°C after which the extracted liquid is separated. The residue is again treated by extraction with ammonia water. Both the first and the second extracted solutions are mixed together and filtered by filter cloth.

Next, the filtered solution of 9.5 liters is concentrated to 150 ml at 40°C under the decompressed condition. The pH of the filtered solution is adjusted to pH 5.4 by means of hydrochloric acid treatment and lyophilization. The brown granules of 4.5 grams of acid polysaccharide are obtained.

The granules obtained in the above extraction treatment have a smell which is distinct and one per cent (W/V) solution thereof showed the degree of pH of 4.5-6.0.

The contents of arsenic and heavy metals in the granules of one gram are measured. The result showed the contents of less than two ppm and thirty ppm, respectively. A group of colon bacilli are atonic and the number of general germs is less than 1000/g.

3

The following diagram shows an extraction treatment of the present invention.

```
              husks of pine nuts
                       ↓
                alcohol washing
                       ↓
              ┌─hot water extraction─┐
              ↓                      ↓
      extracted solution         residue
              ↓                      ↓
      hot water extracted     alkali solution
      substance               extraction
                                     ↓
                              pH observation and
                              arrangement
                       ┌─────────────┴────────────┐
                       ↓                          ↓
              precipitate              supernatant liquid

          (I. alkali solution              │add ethanol
              extraction )
                       ┌──────────────────────────┘
                       ↓                          ↓
              precipitate              supernatant liquid

          (II. alkali solution
               extraction )
```

A testing of the activation function of the extracted acid polysaccharide is as follows:

In order to confirm a physiological activation function of the acid polysaccharide obtained by alkaline solution extraction, the following method and arrangement are applied.

First, a human peripheral blood as a target germ for testing activation function is diluted to double quantity with physiological saline solution. The diluted solution is piled on the layer of Ficoll-Hypague (specific gravity is 1.077) and treated with a centrifugal separator for thirty minutes at 1,500 rpm. The red corpsucal and polymorphonuclear leukocyte fraction are taken out from the lower layer of the mixture.

The red corpsucal and polymorphonuclear leukocyte fraction are washed with physiological saline solution and diluted with blood serums prepared in the first centrifugal separator treatment. The obtained diluted mixture is piled on the layer of Mono-Poly Resolving Medium Ficoll-Hypague (specific gravity is 1.114) and treated with the centrifugal separator for thirty minutes at 1,500 rpm. The intermediate layer comprising polymorphonuclear leukocyte is collected. Next, said intermediate layer is washed twice with a culture fluid of RPMI 1640 of non-blood serum and purified polymorphonuclear leukocyte fraction is obtained as a target germ. The purity of the polymorphonuclear leukocyte is more than 95%.

The target germ obtained on the basis of the foregoing treatments is added with hot water extracted polysaccharide in the Table - I as well as extracted acid polysaccharide I and II by alkali solution to confirm its physiological activation function. As a method of confirmation, Klebanoff and Clark's method (J. Lab. Clin. Med. 89:675 - 686, 1977) was applied after being slightly revised.

Namely, the polymorphonuclear leukocyte of $1 \times 10^6$ pcs. are suspended in the culture fluid of 0.5 ml of non-serum RPMI 1640, said culture fluid containing the substances to be tested of various kinds of concentration (hot water extracted polysaccharide, alkali solution extracted acid polysaccharide I and II, and TAK, CM-TAK for comparison) as well as $8 \times 10^{-6}$ M radioactivity $Na^{125}$ I (I $\mu$Ci/ml). An incubation

4

treatment of one hour is carried out at a temperature of 37°C, and followed by a washing with physiological saline solution. Then, adding iced 5% trichloro acetic acid. It is left in an ice bath for twenty minutes, then treated with a centrifugal separator for ten minutes at 3,000 rpm and radioactivity existing in the precipitate (acid insoluble fraction) is measured with gamma-ray counter.

Table - I

| Activity function of polymorphonuclear leukocyte of human peripheral blood by acid polysaccharide extracted from husks of pine nuts. | | | |
|---|---|---|---|
| kind | extract | radioactive iodine taken into acid insoluble fraction of polymorphonuclear leukocyte | |
| | | (pmol/$10^7$ cells/hr) conc. ($\mu$g/ml) | polymorph. |
| husks of pine nuts | hot water extract polysaccharide | 100 1000 | 231 460 |
| husks of pine nuts | alkali solution extract polysaccharide I | 100 1000 | 451 279 |
| ditto | ditto II | 100 1000 | 242 369 |
| T A K | | 100 1000 | 32 89 |
| C M - T A K | | 100 1000 | 6 7 |
| physiological saline solution for comparison | | - - | 7 9 |
| Remarks: (1) T A K, C M - T A K are anti tumour polysaccharide developed in Japan providing with the strongest activity function. Remarks: (2) T A K... ... ... (1 → 3) - beta - D - glucan) (Cancer Res 38, 379-383, 1987) isolated from Alcalignes faecalis var. myxogenes ( I F O 13140). C M - T A K... ... ... (Carboxy-methylised T A K) (Europ. J. Cancer. 15, 211-215, 1979). | | | |

As shown in the Table - I, the acid polysaccharide obtained from alkali solution extraction method of the present invention activates polymorphonuclear leukocyte strongly, and it was shown that the acid polysaccharide obtained from the present invention can provide an activation function surpassing T A K, C M - T A K which have been considered to be the strongest of the currently existing anti-tumour polysaccharide in Japan.

Next, in order to provide an anti-contagion medicine in which granules of said acid polysaccharide obtained from the aforementioned treatments is used as principal component the following processes of (1) and (2) are applied.

(1) The pine nuts oil is added to 2 mgs of granules of acid polysaccharide, then gelatinized and made a capsul tablet. (2) 10 grams of the acid polysaccharide are added to approximately 85 ml of water and stirred and dissolved for thirty minutes at temperature between 50 - 60°C. Next, 0.7 gram salt and water is added dissolved solution and 100 ml solution was prepared.

Then the solution was treated by a centrifugal separator for twenty minutes at 10,000 rpm. Insoluble substances are removed and sterilized in a high pressure autoclave. Finally, a sterilized ampul injection is obtained.

Next, the activity of the anti-contagion medicine obtained from the aforementioned treatments is observed by the following testing method.

For measuring the activity of the anti-contagion medicine of the acid polysaccharide the following (1) and (2) testings are applied.

(1) Effectiveness testing against an infection of colon bacilli:

The granules of 0.25 mg or the physiological saline solution is prescribed for ddy mice (five-week year, male), each group comprising ten to twelve mice. On the second day colon bacilli drug of $4 \times 10^6$ are transplanted into abdomen of the mice and the number of surviving mice is counted after twenty four hours.

(2) Effectiveness testing against viral infectious disease:

10 mg/kg of said infection liquid is injected the designated times in the muscle of the thigh or in the middle part of the scapula of backbone of dogs and cats infected with viral disease, and change with the passage of time of general status and the number of white blood corpuscle in the blood is observed.

Obstruction activity against infection of colon bacilli in mice and relaxation status of disease of cats suffering from viral disease are taken as indexes and working effects of anti-contagion medicine is observed. The results are as follows:

(1) Effectiveness against infection of colon bacilli:

As shown in the Table - II, if abdomens of mice are previously treated with ammonia solution extract, said extract containing granules that are dissolved in the physiological saline solution, infection of colon bacilli was remarkably inhibited.

Table - II

| Defend-effect of ammonia water extract of pine nuts (granular acid polysaccharide) against colon bacilli infection. | | |
|---|---|---|
| medicine | | number of mice alive after five days |
| alkali solution extract of husks of pine nuts | I | 7/10 (70%) |
| | II | 5/10 (50%) |
| physiological saline solution for comparison | | 1/12 (8%) |

(2) Effectiveness against infection of viral disease:

Ammonia water extract of the husks of pine nuts is hypodermically injected near the scapula of backbone or intermuscularly injected into the femoral region of cats and dogs. As a result viral diseases such as Calici viral glossitis, stomatitis, toothtis, Parvo viral cats leukopenia and Herpes viral nose-bronchitis are greatly inhibited, promoting appetite and improving general status. Some of the animals fully recovered from the disease. To the contrary, the medicine was ineffective against disease such as mammary cancer or fibrous sarcoma that the virus does not cause. Table - III shows results against viral diseases.

Table - III  Defend-effect of acid polysaccharide (injection) extracted from pine nuts against viral disease.

| animal | year month | m. or f. | name of disease | status | amount mg/kg | method | result |
|---|---|---|---|---|---|---|---|
| cat | 2 | f. | Calici viral infection | cough, sneeze | 10 | H × 2 | + relaxation |
| | 2 | f. | ditto | stomatitis | 10 | H × 2 | + ditto |
| | 2 | f. | ditto | snivel | 10 | H × 2 | + ditto |
| | 2 | f. | ditto | fever | 10 | H × 2 | + ditto |
| | 2 | m. | ditto | no appetite | 10 | H × 1 | +++ fully recovered after 24 hrs. |
| | 12 | m. | ditto | glossitis | 10 | M × 3 | ++ recovered |
| | 12 | m. | ditto | ditto | 10 | M × 3 | ++ recovered |
| | 2 | f. | Herpes viral bronchitis | cough, sneeze, nasitis, fever | 10 | M × 2 | ++ recovered |
| | 2 | f. | ditto | snivel | 40 | H × 1 | ++ recovered |
| | 12 | m. | ditto | gum | 10 | M × 2 | ++ recovered |
| | 3 | m. | Parvo viral leukocytepenia | leukocytepenia, vomiting | 10 | M × 2 | ++ recovered incre. corpuscle |
| dog | 2 | m. | ditto | ditto | 10 | H × 2 | +++ fully recovered after 24 hrs. |
| cat | 72 | m. | fibrous cancer | | 10 | M × 15 | - no effect |
| dog | 12 | f. | mammary cancer | | 10 | H × 2 | - no effect |

m.:male    f.:female    H.:hypodermic injection    M.:muscle injection

As aforementioned, the present invention relates to a method of extracting an acid polysaccharide of high activity from the husks of pine nuts. The inventors successfully developed an effective scope of utilization of the extract. The method presents the following technical advantage.

(1) It became possible to extract a substance of high physiological activity from the husks of pine nuts which have so far been neglected.

(2) Alkali solution is used as a means of extraction. It is therefore possible to repeat the means without trouble and accordingly a valuable component is effectively extracted.

(3) The substance extracted and recovered by the present invention is an acid polysaccharide that has an activation function of polymorphonuclear leukocyte. This kind of acid polysaccharide has not been heretofore used as a substance which promotes activity of polymorphonuclear leukocyte. The polymorphonuclear leukocyte has a function to defend infection. It is also possible that the invention can be used as medicine, making use of its activity promotion function.

In particular, as in aforementioned experiments, infection of colon bacilli to mice is inhibited and viral disease of cats and dogs is effectively controlled.

Furthermore, as the anti-contagion medicine of the present invention is extracted and recovered from the husks of natural pine nuts, it does not produce any strong adverse effects which have been seen in other anti-virus drugs. It is also possible to combine it with other curative means or anti-virus drug to effectively cure various viral diseases.

## Claims

1. A method of extracting a physiological active substance in which a granular acid polysaccharide is a principal component, comprising the steps of
immersing the husks of pine nuts in an alkaline solution, proceeding with an extraction treatment by the alkali solution and obtaining an extracted solution,
separating an insoluble substance by filtrating treatment of the extracted solution,
adjusting the degree of pH after the extracted solution obtained by the separating treatment has been concentrated, and proceeding with a lyophilization treatment of said extracted solution to obtain said physiological active substance in which a granular acid polysaccharide is a principal component.

2. A method of extracting a physiologically active substance comprising a granular acid polysaccharide component as a principal component, said granular acid polysaccaride component being obtained by extraction from husks of white pine nuts (pinus pentaphylla), the extraction method comprising the steps of
a) removing husks from the white pine nuts,
b) immersing the husks in an alkaline solution,
c) extracting the husks by treatment with said alkaline solution,
d) adjusting the pH of extracted solution,
e) precipitating said extract by means of centrifugal separator,
f) separating any insoluble substance from said extract by filtration treatment,
g) concentrating said extract,
h) dialysing with ethanol said concentrate, where the pH of said concentrate is adjusted with diluted hydrochloric acid to a pH value of 5.4, and
i) lyphophilizing to obtain said granular acid polysaccharide component.

3. A method of claim 1 or 2, characterized in that the husks are removed by means of a shell cracking apparatus.

4. A method of any of claims 1-3, characterized in that the husks are immersed in an alkali solution of pH 7.5 to 10, the amount of said alkali solution being five times that of said husks.

5. A method of any of claims 1-4, characterized in that the adjustment of pH occurs with hydrochloric acid.

6. An anti-contagion medicine in which an acid polysaccharide obtained by the extraction methods as defined in any one of claims 1 - 5 is used as an effective ingredient.

7. A medicine according to claim 6 for use as an anti-viral, antibacterial or anti-herpes medicine.

8. The acid polysaccharide obtained by the extraction methods as defined in any one of claims 1 - 5 for use as a polymorphonuclear leukocyte activating medicine.

EP 0 395 757 B1

**Patentansprüche**

**1.** Verfahren zum Extrahieren einer physiologisch aktiven Substanz, in der ein granulares saures Polysaccarid die Hauptkomponente ist, wobei folgende Schritte vorgesehen sind:

Eintauchen der Schalen von Fichten- oder Pinienkernen in eine Alkalilösung,

Fortschreiten mit einer Extraktionsbehandlung durch die Alkalilösung und Erhalt einer extrahierten Lösung,

Trennen einer nichtlöslichen Substanz durch Filterbehandlung der extrahierten Lösung,

Einstellung des pH-Wertes nachdem die durch die Trennbehandlung erhaltene extrahierte Lösung konzentriert wurde, und

Fortschreiten mit einer Lyophilisierungsbehandlung (Gefriertrocknung) der extrahierten Lösung zum Erhalt der physiologisch aktiven Substanz in der ein granulares saures Polysaccarid eine Hauptkomponente ist.

**2.** Verfahren zum Extrahieren einer physiologisch aktiven Substanz, die als Hauptkomponente eine granulare saure Polysaccarid-Komponente aufweist, wobei die granulare saure Polysaccarid-Komponente erhalten wird durch die Extraktion von Schalen der Kerne der Weißfichte oder Weißpinie (pinus pentaphylla), wobei das Extraktionsverfahren die folgenden Schritte vorsieht:

a) Entfernen der Schalen von den Weißfichtenkernen,

b) Eintauchen der Schalen in eine Alkalilösung,

c) Extraktion der Schalen durch Behandlung mit der Alkalilösung,

d) Einstellung des pH-Wertes der extrahierten Lösung,

e) Ausscheidung des Extrakts durch eine Zentrifugentrennvorrichtung,

f) Trennen jedweder nichtlöslichen Substanz von dem Extrakt durch Filtrationsbehandlung,

g) Konzentrieren des Extraktes,

h) Dialysierung des Konzentrats mit Ethanol, wobei der pH- Wert des Konzentrats mit verdünnter Salzsäure auf einen pH-Wert von 5,4 eingestellt wird, und

i) Lyophilisieren zum Erhalt der granularen sauren Polysaccarid-Komponente.

**3.** Verfahren nach Anspruch 1 oder 2, dadurch gekennzeichnet, daß die Schalen durch eine Schalen-Brechvorrichtung entfernt werden.

**4.** Verfahren nach einem der Ansprüche 1 bis 3, dadurch gekennzeichnet, daß die Schalen in einer Alkalilösung von pH 7,5 bis 10 eingetaucht werden, wobei die Menge der Alkalilösung das Fünffache der Schalen ist.

**5.** Verfahren nach einem der Ansprüche 1 bis 4, dadurch gekennzeichnet, daß die Einstellung des pH-Wertes mit Salzsäure erfolgt.

**6.** Anti-Kontagiöses Arzneimittel in dem als ein effektiver Bestandteil ein Säurepolysaccarid verwendet wird, das durch die Extraktionsverfahren gemäß einem der Ansprüche 1 bis 5 erhalten wurde.

**7.** Arzneimittel nach Anspruch 6 zur Verwendung als ein antivirales, antibakterielles oder Anti-Herpes Arzneimittel.

**8.** Säurepolysaccarid erhalten durch die Extraktionsverfahren nach einem der Ansprüche 1 bis 5 zur Verwendung als ein polymorphonukleares Leukozyt-aktivierendes Arzneimittel.

**Revendications**

**1.** Un procédé d'extraction d'une substance physiologiquement active dont un composant principal est un polysaccharide acide granulaire, comprenant les étapes suivantes :

plonger les enveloppes de pignons dans une solution alcaline, procéder à un traitement d'extraction par la solution alcaline et obtenir une solution extraite,

séparer une substance insoluble par un traitement de filtration de la solution extraite,

ajuster la valeur de pH après avoir concentré la solution extraite obtenue par le traitement de séparation, et

procéder à un traitement de lyophilisation de ladite solution extraite pour obtenir ladite substance

9

physiologiquement active dont un composant principal est un polysaccharide acide granulaire.

2. Un procédé d'extraction d'une substance physiologiquement active comprenant un composant polysaccharide acide granulaire comme principal composant, ledit composant polysaccharide acide granulaire étant obtenu par extraction à partir d'enveloppes de pignons de pin blanc (*Pinus pentaphylla*), le procédé d'extraction comprenant les étapes suivantes :
   a) retirer les enveloppes des pignons de pin blanc,
   b) plonger des enveloppes dans une solution alcaline,
   c) extraire les enveloppes par traitement avec ladite solution alcaline,
   d) ajuster le pH de la solution extraite,
   e) précipiter ledit extrait au moyen d'un séparateur centrifuge,
   f) séparer toute substance insoluble dudit extrait par un traitement de filtration,
   g) concentrer ledit extrait,
   h) dialyser ledit concentré avec de l'éthanol, le pH dudit concentré étant adjusté avec de l'acide chlorhydrique dilué à un pH de 5,4, et
   i) lyophiliser pour obtenir ledit composant polysaccharide acide granulaire.

3. Un procédé de la revendication 1 ou 2, caractérisé en ce que les enveloppes sont retirées au moyen d'un appareil à briser les coques.

4. Un procédé de l'une quelconque des revendications 1 à 3, caractérisé en ce que les enveloppes sont plongées dans une solution alcaline de pH 7,5 à 10, la quantité de ladite solution alcaline étant cinq fois supérieure à celle desdites enveloppes.

5. Un procédé de l'une quelconque des revendications 1 à 4, caractérisé en ce que l'adjustement de pH s'effectue avec l'acide chlorhydrique.

6. Un médicament antiinfectieux dans lequel un polysaccharide obtenu par les procédés d'extraction tels que définis dans l'une quelconque des revendications 1 à 5 est utilisé comme ingrédient actif.

7. Un médicament selon la revendication 6, pour son utilisation comme médicament antiviral, antibactérien ou antiherpétique.

8. Le polysaccharide acide obtenu par les procédés d'extraction tels que définis dans l'une quelconque des revendications 1 à 5, pour son utilisation comme médicament activant les granulocytes.